(19) Europäisches Patentamt
European Patent Office
Office européen des brevets

(11) Numéro de publication: **0 120 799**
**A1**

(12) # DEMANDE DE BREVET EUROPEEN

(21) Numéro de dépôt: **84430004.6**

(51) Int. Cl.$^3$: **A 61 K 39/385**

(22) Date de dépôt: **23.02.84**

(30) Priorité: **25.02.83 FR 8303297**

(43) Date de publication de la demande:
**03.10.84 Bulletin 84/40**

(84) Etats contractants désignés:
**AT BE CH DE FR GB IT LI LU NL SE**

(71) Demandeur: **Société Anonyme dite: IMMUNOTECH**
**51 rue Saint Georges**
**F-75009 Paris(FR)**

(72) Inventeur: **Capron, André**
**Villa Maison Blanche**
**F-59133 Phalempin(FR)**

(72) Inventeur: **Delaage, Michel Résidence Pontet-Pascal**
**Bât. 5-3 Rue Paul Eluard**
**F-33600 Pessac(FR)**

(72) Inventeur: **Hirn, Michel**
**Villa Gyptis 359 Corniche Kennedy**
**F-13007 Marseille(FR)**

(72) Inventeur: **Nirde, PHilippe**
**15 Tour de l'Ayguette**
**F-64140 Billiere(FR)**

(72) Inventeur: **De Reggi, Max**
**68 Chemin de la Lune**
**F-38170 Seyssins(FR)**

(74) Mandataire: **Rinuy, Santarelli**
**14, avenue de la Grande Armée**
**F-75017 Paris(FR)**

(54) **Médicament et vaccin pour lutter contre les parasitoses à helminthes, dérivant d'un composé ecdystéroide.**

(57) Le médicament est constitué des immunoglobulines anti-ecdysone ou de leurs fragments anti-ecdysone obtenues à partir d'un conjugué ecdystéroïde-macromolécule et le vaccin est un tel conjugué dans lequel la macromolécule est une protéine, ledit ecdystéroïde étant tout composé analogue à l'ecdysone induisant la formation d'anticorps reconnaissant l'ecdysone.

EP 0 120 799 A1

Croydon Printing Company Ltd.

Médicament et vaccin pour lutter contre les parasitoses à helminthes, dérivant d'un composé ecdystéroïde

La présente invention concerne un médicament et un vaccin pour lutter contre les parasitoses à helminthes.

Parmi les parasitoses à helminthes connues, on sait que la bilharziose est une maladie tropicale qui atteint quelques centaines de millions d'individus. Elle est le fait d'un parasite appartenant à la famille des helminthes (vers), caractérisée par un cycle biologique à deux hôtes : un vertébré (par exemple l'homme) et un mollusque.

L'évolution de la maladie est longue, de 3 à 20 ans, les différentes variétés de parasites affectant plus spécifiquement tel ou tel organe.

Les moyens de lutte contre les parasitoses sont très divers et très insuffisants. On peut distinguer :

- L'action sanitaire et l'hygiène :
  Ces moyens pourraient être très efficaces mais ils présupposent, en fait, le décollage économique des régions concernées avec adduction d'eau, nouvelles habitudes alimentaires, etc. Or les parasitoses elles-mêmes sont un obstacle majeur au développement : outre qu'elles altèrent la capacité de travail des populations concernées, elles exercent un effet dissuasif sur les activités de coopération technique ou le tourisme. C'est donc un cercle vicieux. La destruction des mollusques dans l'eau n'est possible que sur des régions très limitées.
- Au niveau de l'individu, on dispose depuis peu, par exemple d'anti-bilharziens d'une réelle efficacité mais qui laissent encore un pourcentage non négligeable de cas rebelles et, en tout état de cause, ils ne sont administrés qu'après l'apparition des premiers signes cliniques. Leur diffusion à grande échelle dans le Tiers-Monde pose encore des problèmes d'ordre sanitaire et économique.
- La recherche de vaccins anti-parasitaires a marqué le pas jusqu'ici. Outre que les parasites sont dotés de capacités remarquables pour se protéger du système immunitaire, on ne sait faire que des vaccins excessivement coûteux, soit à partir de protéines naturelles extraites

de parasites en culture (quand la culture est possible), soit de façon plus prospective à partir de peptides de synthèse.

La présente invention vise un moyen de lutte contre les parasitoses à helminthes comme par exemple la bilharziose.

On sait déjà que, par couplage entre l'ecdysone ou 2$\beta$, 3$\beta$, 14$\alpha$, 22R, 25-penta-hydroxy-5$\beta$-cholest-7-ene-6-on (hormone de mue des artropodes) ou la 20-hydroxy-ecdysone, avec une macromolécule telle une protéine, on obtient un conjugué qui, injecté à un lapin, provoque la formation d'anti-corps capables de neutraliser l'ecdysone. Cette propriété est mise à profit pour le dosage radio-immunologique de l'ecdysone ou pour provoquer un effet antagoniste de l'ecdysone dans les systèmes de cultures de tissus.

Toutefois, rien de ces connaissances antérieures ne pouvait suggérer ou laisser supposer la possibilité de lutter contre les parasites au moyen de tels anticorps. En effet, la demanderesse a trouvé de façon surprenante et inattendue, d'une part, que l'administration d'anticorps anti-ecdysone était capable de bloquer le développement d'un parasite de type helminthe chez son hôte définitif (mammifère) et, d'autre part, que la production de ces anticorps par une vaccination à l'aide d'un conjugué ecdystéroïde-protéine entraînait la protection des sujets sains et la guérison des sujets parasités.

L'invention ouvre ainsi une voie nouvelle dans la thérapeutique anti-parasitose à helminthes, par exemple anti-bilharziose, en fournissant un nouveau médicament ainsi qu'un nouveau vaccin.

Le médicament selon l'invention est caractérisé par le fait qu'il est constitué des immunoglobulines anti-ecdysone ou de leurs fragments anti-ecdysone obtenues à partir d'un conjugué ecdystéroïde-macromolécule et : soit

extraites du sérum d'un animal immunisé avec ce conjugué, soit produites par un hybridome obtenu à partir descellules d'un animal immunisé, soit produites par des cellules modifiées par manipulation génétique.

Le vaccin selon l'invention est essentiellement constitué par un conjugué ecdystéroïde-macromolécule dans lequel ladite macromolécule est avantageusement une protéine.

On entend dans la présente description par conjugué ecdystéroïde-macromolécule, le produit résultant du couplage de façon covalente entre un support constitué par une macromolécule et un composé ecdystéroïde.

Suivant d'autres caractéristiques :

- La macromolécule du conjugué ecdystéroïde-macromolécule entrant dans l'élaboration du médicament est de nature quelconque ;
- La macromolécule protéique entrant dans la constitution du vaccin peut être de nature quelconque. Ce peut être, par exemple, l'albumine du sérum (d'origine humaine, bovine, etc) ou une autre protéine, de préférence de haut poids moléculaire, comme les immunoglobulines, les hémocyanines, etc, ou même un polymère synthétique tel que la polylysine ;
- Le composé ecdystéroïde peut être l'ecdysone de formule :

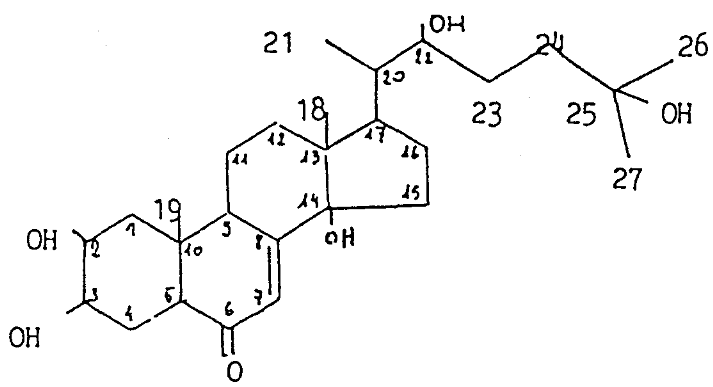

ou l'un de ses dérivés tel que les 2. hémi-succinate ; 3. hémi-succinate ; 22. hémi-succinate ; 6. carboxyméthyl oxime de même que les analogues très proches tels que la

20-hydroxy-ecdysone (ou ecdystérone), l'inokostérone qui diffère de l'ecdysone par l'hydroxylation de la chaîne latérale en position 26 au lieu de 25 et, dans ce cas, c'est le dérivé carboxylique en 26 qui sert au couplage mais, de façon générale, ce composé peut être tout analogue induisant la formation d'anticorps reconnaissant l'ecdysone.

Suivant une variante et pour renforcer l'efficacité vaccinale, on peut utiliser comme composé actif un composé tel que défini ci-dessus dans lequel le support protéique est en outre couplé à un adjuvant de l'immunité comme le muramyl dipeptide.

De façon avantageuse, le couplage du composé ecdystéroïde sur le support protéique est réalisé par l'une des positions 2,3,22,6 ou 26.

De façon également avantageuse, le produit de ce couplage présente un rapport pondéral ecdystéroïde/protéine de 2 à 15 %.

Les exemples suivants feront mieux ressortir la portée et l'intérêt de l'invention.

Exemple d'obtention d'un vaccin selon l'invention :

- 3,2 mg de succinyl ecdystérone sont dissous dans 300 µl de diméthylformamide à température ambiante ;
- On ajoute alors 30 µl de triéthylamine au 1/11e dans la diméthylformamide ;
- A froid, on introduit 30 µl d'éthylchloroformate au 1/16e dans la diméthylformamide dans le milieu réactionnel et on laisse l'activation se poursuivre pendant 12 minutes ;
- Après avoir soustrait 50 µl destinés au couplage au tyrosine méthylester, on ajoute aux 310 µl restants 6 mg de sérum à albumine lyophilisée. L'incubation est alors maintenue une demi-heure.
- Le conjugué obtenu est purifié sur une colonne de G25 SEPHADEX (longueur 10 cm, diamètre 1,5 cm) équilibrée avec du tampon phosphate de Na $10^{-2}$ M, NaCl 1,5 $10^{-2}$M pH 7. Sur ce type de colonne, le conjugué est recueilli dans le volume mort. Les fractions contenant le conjugué sont

réunies et dialysées pendant 36 heures contre deux fois 1 litre de $H_2O$ distillée afin d'éliminer les sels. Après dialyse, on enregistre un spectre U.V. afin de calculer le rendement du couplage. Enfin, le produit est lyophilisé et conservé à l'abri de l'humidité.

Ce produit peut être dilué au moyen de sérum physiologique et injecté directement à l'homme ou l'animal selon les protocoles de vaccination classiques.

<u>Exemple de médicament selon l'invention</u> :

Les immunoglobulines obtenues par immunisation d'un lapin au moyen d'un conjugué tel que défini ci-dessus sont purifiées par précipitation au sulfate d'ammonium puis dialysées et chromatographiées sur protéine A. Après élution à pH 2,4, les immunoglobulines sont neutralisées et lyophilisées.

Elles peuvent être administrées après redissolution dans le sérum physiologique par injection intra-veineuse.

<u>Effets pharmacologiques et toxicologiques</u> :

Les tests effectués ont montré que le principe actif (anticorps anti-ecdysone) n'interférait avec aucune fonction physiologique, son action étant spécifique du parasite.

Le médicament selon l'invention présente à forte dose ou en cas d'injections répétées, une toxicité analogue à celle des immunoglobulines déjà utilisées comme médicament (anti-tétanique par exemple).

Le vaccin selon l'invention est bien toléré et induit une protection efficace et durable.

Le médicament et le vaccin selon l'invention se sont avérés particulièrement efficaces dans les cas de bilharziose.

Il va de soi que l'invention n'a été décrite qu'à titre purement explicatif et nullement limitatif et que toute modification utile pourra y être apportée sans sortir de son cadre.

REVENDICATIONS

1. Médicament et vaccin pour lutter contre les parasitoses à helminthes, caractérisés par le fait qu'ils sont constitués de produits dérivant d'un composé ecdystéroïde.

2. Vaccin selon la revendication 1, caractérisé par le fait qu'il est constitué d'un conjugué ecdystéroïde-macromolécule résultant du couplage covalent entre une macromolécule et au moins un composé ecdystéroïde.

3. Médicament selon la revendication 1, caractérisé par le fait qu'il est constitué par des immunoglobulines anti-ecdysone ou par leurs fragments anti-ecdysone obtenues à partir d'un conjugué selon la revendication 2 et : soit extraites du sérum d'un animal immunisé avec ce conjugué, soit produites par un hybridome obtenu à partir des cellules d'un animal immunisé, soit produites par des cellules modifiées par manipulation génétique.

4. Vaccin ou médicament selon l'une quelconque des revendications 1 à 3, caractérisé par le fait que la macromolécule conjuguée à l'ecdystéroïde est avantageusement une protéine.

5. Vaccin ou médicament selon l'une quelconque des revendications 1 à 4, caractérisé par le fait que ladite protéine est choisie parmi les suivantes : sérum albumine (d'origine humaine ou animale), protéines de préférence de haut poids moléculaire du type immunoglobulines, hémocyanines et analogues, polymères synthétiques du type polylysine et analogues.

6. Vaccin ou médicament selon l'une quelconque des revendications 1 à 5, caractérisé par le fait que le ou les composés ecdystéroïdes couplés induisent la formation d'anticorps reconnaissant l'ecdysone.

7. Vaccin ou médicament selon l'une quelconque des revendications 1 à 6, caractérisé par le fait que les composés ecdystéroïdes sont couplés à la macromolécule par l'une des positions 2, 3, 6, 22 ou 26.

8. Vaccin ou médicament selon l'une quelconque des revendications 1 à 7, caractérisé par le fait que le ou les composés ecdystéroïdes couplés à la macromolécule est l'un des composés suivants :

- ecdysone de formule :

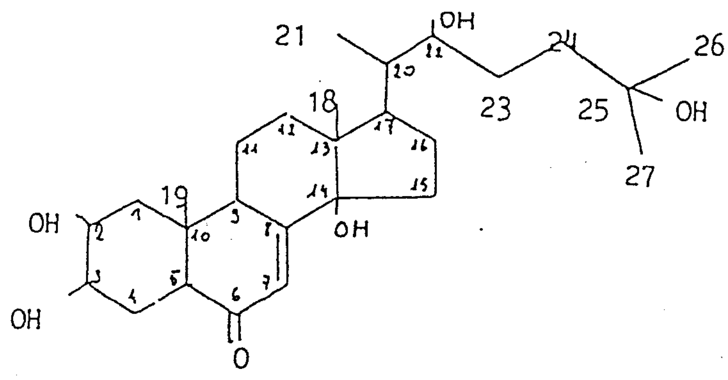

- 20-hydroxy-ecdysone (ou ecdystérone) ;
- inokostérone (qui dérive de l'ecdysone par l'hydroxylation de la chaîne latérale en position 26 au lieu de 25) ;
- les dérivés des trois composés ci-dessus, à savoir les 2.hémi-succinates ; 3.hémi-succinates ; 22.hémi-succinates ; 6.carboxyméthyl-oxymes et analogues.

9. Vaccin ou médicament selon l'une quelconque des revendications 1 à 8, caractérisé par le fait que la macromolécule est de plus couplée à un adjuvant connu de l'immunité.

10. Vaccin ou médicament selon la revendication 9, caractérisé par le fait que ledit adjuvant de l'immunité est du type muramyl-dipeptide.

11. Vaccin ou médicament selon l'une quelconque des revendications 1 à 10, caractérisé par le fait que le produit de couplage présente un rapport pondéral de 2 à 15 %.

12. Vaccin ou médicament selon l'une quelconque des revendications 1 à 11 appliqué à la lutte contre la bilharziose.

Office européen des brevets

**RAPPORT DE RECHERCHE EUROPEENNE**

0120799
Numéro de la demande

EP 84 43 0004

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X | CHEMICAL ABSTRACTS, vol. 93, no. 11, 15 septembre 1980, page 356, no. 110085j, Columbus, Ohio, USA D.T. HUNG et al.: "Preparation of an ecdysone immunogen for radioimmunoassay work" & J. BIOL. CHEM. 1980, 255(13), 6047-6048 * Abrégé * --- | 1 | A 61 K 39/385 |
| A | FR-A-2 318 877 (M. DELAAGE et al.) * Page 3, lignes 10-16 * ----- | 1-12 | |
| | | | **DOMAINES TECHNIQUES RECHERCHES (Int. Cl. 3)** |
| | | | A 61 K |

Le présent rapport de recherche a été établi pour toutes les revendications

| Lieu de la recherche | Date d'achèvement de la recherche | Examinateur |
|---|---|---|
| LA HAYE | 30-05-1984 | REMPP G.L.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant